# EUROPEAN PATENT APPLICATION

(11) **EP 2 484 771 A1**
(43) Date of publication of application: **08.08.2012**
(21) Application number: 12166567.3
(22) Date of filing: 18.03.2008
(51) Int. Cl.: C12P 7/56

(54) **Process for producing D-lactic acid from glycerol employing Serratia marcescens**

(30) Priority: 19.03.2007 JP 2007071532; 14.05.2007 JP 2007128432; 01.06.2007 JP 2007147363; 02.08.2007 JP 2007202270; 21.11.2007 JP 2007302158; 21.11.2007 JP 2007302157
(62) Divisional of application: 08738854.2
(71) Applicant: Sumitomo Chemical Company Limited, Tokyo 104-8260 (JP)
(72) Inventor: Kishimoto, Kazuhisa, Osaka, 576-0016 (JP)
(74) Representative: Vossius & Partner

(57) **Abstract**

There is provided is a method for producing lactic acid from glycerol, which comprises culturing a specific bacterium capable of producing lactic acid in a culture medium containing glycerol, or making cells of the bacterium, processed cells of the bacterium or immobilized product thereof contact with glycerol.

## Description

### TECHNICAL FIELD

The present invention relates to a method for producing lactic acid from glycerol using a microorganism.

### BACKGROUND ART

Bio Diesel Fuel (BDF) is a carbon-neutral alternative fuel for light oil, and has recently been receiving attention as a fuel contributing to resolving environmental problems such as depletion of energy resources, global warming, and air pollution.

Bio Diesel Fuel is produced from vegetable oils, animal fats, waste oil, and the like. In this time, a waste liquid containing glycerol is produced as a by-product. There is no specific use for the waste liquid, so that it is currently just discarded.

As a method for utilizing Bio Diesel waste, Japanese Unexamined Patent Publication (Kokai) No. 2006-180782 discloses a method for producing hydrogen and ethanol from glycerol in the waste using a bacterium of the genus Enterobactor.

Regarding production of organic acids, Japanese Unexamined Patent Publication (Kokai) No. 2003-235592 discloses a method for producing organic acid from glucose as a carbon source, using aerobacter (particularly, coryneform bacteria). Japanese Unexamined Patent Publication (Kokai) No. 2004-501634 discloses a bacterial strain (Mannheimia sp. 55E) which produces various organic acids (containing lactic acid) from glucose.

It has been known that there are several species of bacterium which can produce lactic acid from glycerol. For examples, European Patent Application EP 0 361 082 A2, Ke-Ke Cheng et al., Biotechnology Letters (2004)26: 911-915, F. Barbirato et. al. , Appl Microbiol Biotechnol (1995) 43:786-793, and T. Homann et al., Appl Microbiol Biotechnol (1990) 33: 121-126 describe bacteria which can produce lactic acid from glycerol. In addition, European Patent Application EP 0 361 082 A2 discloses that Citrobacter freundii and Klebsiella pneumoniae produce D-lactic acid from glycerol under an anaerobic condition. In any literatures, the amount of lactic acid or D-lactic acid produced from glycerol was still unsatisfactory. Therefore, introduction of a microorganism has been expected, which has higher production capacity of lactic acid or D-lactic acid. It is also required to develop a method, which enables production of D-lactic acid at low cost by a simply manner. There is no description which indicates the following bacteria can produce lactic acid from glycerol: a bacterium of a genus selected from the bacterial genus group consisting of Agrobacterium, Pseudomonas, Achromobacter, Devosia, Frateuria, Paenibacillus, Brevibacterium, Hafnia, Raoultella, Rhizobium and Serratia; a bacterium of a species selected from the bacterial species group consisting of Arthrobacter nicotianae, Arthrobacter protophormiae, Streptomyces fimicarius, Streptomyces alboflavus and Streptomyces althioticus; a bacterium of a genus selected from the bacterial genus group consisting of Nocardioides and Proteus; and a bacterium of a species selected from the bacterial species group consisting of Arthrobacter aurescens, Arthrobacter citrous, Bacillus badius, Bacillus sphaericus, Nocardia uniformis, Streptomyces carnosus and Streptomyces cellulosae.

In genetic recombinants of Escherichia coli, it is known that there are recombinants which can produce D-lactic acid from carbon sources other than glycerol. However, it has not been known that there is a recombinant of Escherichia coli which can produce D-lactic acid from glycerol in high optical purity.

### DISCLOSURE OF INVENTION

It is desired to produce useful substances from the Bio Diesel waste for effective utilization of resources.

An object of the present invention is to provide a method for producing D-lactic acid from glycerol in high optical purity by a simple manner, by culturing in a culture medium containing glycerol a bacterium of a genus selected from the bacterial genus group consisting of Agrobacterium, Pseudomonas, Achromobacter, Devosia, Frateuria, Paenibacillus, Brevibacterium, Hafnia, Raoultella, Rhizobium and Serratia; a bacterium of a species selected from the bacterial species group consisting of Arthrobacter nicotianae, Arthrobacter protophormiae, Streptomyces fimicarius, Streptomyces alboflavus and Streptomyces althioticus; or a bacterium of Escherichia coli ATCC 700926 strain (hereinafter, these bacteria are collectively referred to as D-lactic acid-producing bacteria used for the present invention or simply as D-lactic acid-producing bacteria), particularly under an aerobic condition, or by contacting to glycerol cells of the bacterium, processed cells of the bacterium or immobilized product thereof. Further object of the present invention is to provide a method for producing lactic acid from glycerol in high conversion rate by a simple manner, by culturing a bacterium of a genus selected from the bacterial genus group consisting of Nocardioides and Proteus; or a bacterium of a species selected from the bacterial species group consisting of Arthrobacter aurescens, Arthrobacter citrus, Bacillusbadius, Bacillus sphaericus, Nocardia uniformis, Streptomyces carnosus and Streptomyces cellulosae (hereinafter, these bacteria are collectively referred to as lactic acid-producing bacteria used for the present invention, or simply as lactic acid-producing bacteria), particularly under an aerobic condition, or by contacting to glycerol cells of the bacterium, processed cells of the bacterium or immobilized product thereof.

Namely, the present invention can provide the followings:
1. A method for producing lactic acid from glycerol, which comprises culturing any one of the following bacteria in a culture medium containing glycerol or contacting cells of the bacterium, processed cells of the bacterium or immobilized product thereof with glycerol:
   (a) a bacterium belonging to a genus selected from the bacterial genus group consisting of Agrobacterium, Pseudomonas, Achromobacter, Devosia, Frateuria, Paenibacillus, Brevibacterium, Hafnia, Raoultella, Rhizobium and Serratia, and being capable of producing D-lactic acid from glycerol in about 70%e.e. or higher optical purity;
   (b) a bacterium belonging to a species selected from the bacterial species group consisting of Arthrobacter nicotianae, Arthrobacter protophormiae, Streptomyces fimicarius, Streptomyces alboflavus and Streptomyces althioticus, and being capable of producing D-lactic acid from glycerol in about 70%e.e. or higher optical purity;
   (c) a bacterium of Escherichia coli ATCC 700926 strain;
   (d) a bacterium belonging to a genus selected from the bacterial genus group consisting of Nocardioides and Proteus, and being capable of producing lactic acid from glycerol; and
   (e) a bacterium belonging to a species selected from the bacterial species group consisting of Arthrobacter aurescens, Arthrobacter citreus, Bacillus badius, Bacillus sphaericus, Nocardia uniformis, Streptomyces carnosus and Streptomyces cellulosae, and being capable of producing lactic acid from glycerol;
2. A method for producing D-lactic acid from glycerol, which comprises:
   culturing in a culture medium containing glycerol, a bacterium belonging to a genus selected from the bacterial genus group consisting of Agrobacterium, Pseudomonas, Achromobacter, Devosia, Frateuria, Paenibacillus, Brevibacterium, Hafnia, Raoultella, Rhizobium and Serratia, and being capable of producing D-lactic acid from glycerol in about 70%e.e. or higher optical purity; or
   contacting cells of the bacterium, processed cells of the bacterium or immobilized product thereof with glycerol;
3. The method according to the above 2, wherein a bacterium belonging to a genus selected from the bacterial genus group consisting of Agrobacterium, Pseudomonas, Achromobacter, Devosia, Frateuria, Paenibacillus, Brevibacterium, Hafnia, Raoultella, Rhizobium and Serrtia is a bacterium of Agrobacterium radiobacter, Pseudomonas auricularis, Pseudomonas azotaformans, Pseudomonas chlororaphis, Pseudomonas taetrolens, Pseudomonas fragi, Pseudomonas sp. , Achromobacter denitrificans, Devosia riboflavina, Frateuria aurantia, Paenibacillus validus, Brevibacterium butanicum, Hafnia alvei, Raoultella planticola, Raoultella terrigena, Rhizobium radiobacter or Serratia marcesceus;
4. The method according to the above 3, wherein a bacterium belonging to a genus selected from the bacterial genus group consisting of Agrobacteri um, Pseudomonas, Achromobacter, Devosia, Frateuria, Paenibacillus, Brevibacterium, Hafnia, Raoultella, Rhizobium and Serratia is a bacterium of Agrobacterium radiobacter FERM BP-3843 strain, Pseudomonas auricularis NBRC 13334 strain, Pseudomonas azotoformans JCM 277'7 strain, Pseudomonas chlororaphis NBRC 3521 strain, Pseudomonas taetrolens NBRC 3460 strain, Pseudomonas fragi JCM 20552 strain, Pseudomonas species ATCC 53617 strain, Achromobacter denitrificans NARC 12669 strain, Achromobacter denitrificans ATCC 35699 strain, Devosia, riboflavina NBRC 13584 strain, Frateuria aurantia NARC 3247 strain, Paenibacillus validus NARC 13635 strain, Brevibacterium butanicum ATCC 21196 strain, Hafnia alvei NBRC 3731 strain, Raoultella planticola JCM 7251 strain, Raoultella terrigena JCM 1687 strain, Rhizobium radiobacter NARC 13532 strain or Serratia marcesceus NBRC 12648 strain;
5. A method for producing u-lactic acid from glycerol, which comprises:
   culturing in a culture medium containing glycerol, a bacterium belonging to a species selected from the bacterial species group consisting of Arthrobacter nicotianae, Arthrobacter protophormiae, Streptomyces fimicarius, Streptomyces albaflavus and Streptomyces althioticus, and being capable of producing D-lact3c acid from glycerol in about 70%e.e. or higher optical purity; or
   contacting cells of the bacterium, processed cells of the bacteriim or immobilized product thereof with glycerol;
6. The method according to the above 5, wherein a bacterium belonging to species selected from the bacterial species group consisting of Arthrobacter nicotianae, Arthrobacter protophormiae, Streptomyces fimicarius, Streptomyces alboflavus and Streptomyces althioticus is a bacterium of Arthrobacter nicotianae JCM 1333 strain, Arthrobacter protophormiae JCM 1973 strain, Streptomyces fimicarius ATCC 21900 strain, Streptomyces alboflavus NBRC 13196 strain or Streptomyces althioticus NARC 15956 strain;
7. A method for producing D-lactic acid from glycerol, which comprises:
   culturing in a culture medium containing glycerol, a bacterium of Escherichia coli ATCC 700926 strain, or
   contacting cells of the bacterium, processed cells of the bacteriim or immobilized product thereof with glycerol;
8. The method according to any one of the above 2 to 7, further comprises recovering D-lactic acid from a culture obtained by culturing;
9. A method for producing lactic acid from glycerol, which comprises:
   culturing in a culture medium containing glycerol, a bacterium belonging to a genus selected from the bacterial genus group consisting of Nocardioides and Proteus, and being capable of producing lactic acid from glycerol; or
   contacting cells of the bacterium, processed cells of the bacteriim or immobilized product thereof with glycerol;
10. The method according to the above 9, wherein a bacterium belonging to a genus selected from the bacterial genus group consisting of Nocardioides and Proteus is a bacterium of Nocardioides simplex or Proteus vulgaris;
11. The method according to the above 10, wherein a bacterium belonging to a genus selected from the bacterial genus group consisting of Nocardioides and Proteus is a bacterium of Nocardioides simplex NBRC 12069 strain or Proteus vulgaris NBRC 3851 strain;
12. A method for producing lactic acid from glycerol, which comprises:
   culturing in a culture medium containing glycerol, a bacterium belonging to a species selected from the bacterial species group consisting of Arthrobacter aurescens, Arthrobacter citreus, Bacillus badius, Bacillus sphaericus, Nocardia uniformis, Streptomyces carnosus and Streptomyces cellulosae, and being capable of producing lactic acid from glycerol; or
   contacting cells of the bacterium, processed cells of the bacteriim or immobilized product thereof with glycerol;
13. The method according to the above 12, wherein a bacterium belonging to a species selected from the bacterial species group consisting of Arthrobacter aurescens, Arthrobacter citreus, Bacillus badius, Bacillus sphaericus, Nocardia uniformis, Streptomyces carnosus and Streptomyces cellulosae is a bacterium of Arthrobacter aurescens NBRC 12136 strain, Arthrobacter citreus NBRC 12957 strain, Bacillus badius ATCC 14574 strain, Bacillus sphaericus NBRC 3341 strain, Nocardia uniformis NBRC 13702 strain, Streptomyces carnosus NBRC 13025 strain or Streptomyces cellulosae NARC 3713 strain;
14. The method according to any one of the above 9 to 13, further comprises recovering lactic acid from a culture obtained by culturing;
15. The method according to any ore of the above 1 to 14, wherein the bacterium is cultured under anaerobic condition; and
16. The method according to any one of the above 1 to 15, wherein glycerol is originated from Bio Diesel waste.

### MODE FOR CARRYING OUT THE INVENTION

The D-lactic acid-producing bacteria used for the present invention are specifically exemplified by the bacterial genera, bacterial species and bacterial strains described in the above 2 to 7. Among these D-lactic acid-producing bacteria, preferred bacteria are those being able to produce D-lactic acid from glycerol in higher conversion rate. Examples of such bacteria include a Brevibacterium butanicum ATCC 21196 strain, Devosia ribaflavzna NBRC 13584 strain, Escherichia col i ATCC 700926 strain, Frateuria aurantia NBRC 3247 strain, Serratia marcesceus NBRC 12648 strain, Agrobacterium radiobacter FERM BP-3843 strain, Rhizobium radiobacter NBRC 13532 strain, Pseudomonas auricularis NARC 13334 strain, Pseudomonas azotoformans JCM 2777 strain, Pseudomonas, chlororaphis NBRC 3521 strain, Pseudomonas taetrolens NBRC 3460 strain, Pseudomonas fragi JCM 20552 strain, Pseudomonas species ATCC 53617 strain, Paenibacillus validus NBRC 13635 strain, Achromobacter denitrificans NARC 12669 strain, Achromobacter denitrificans ATCC 35699 strain, Streptomyces fimicarius ATCC 21900 strain, Streptomyces alboflavus NBRC 13196 strain, Streptomyces althioticus NBRC 15956 strain, Hafnia alvei NBRC 3731 strain, Arthrobacter nicotianae JCM 1333 strain, Arthrobacter protophormiae JCM 1973 strain, Raoultella planticola JCM 7251 strain and Raoultella terrigena JIM 1687 strain, but they are not limited thereto. Escherichia coli ATCC 700926 strain, Serratia marcesceus NBRC 12648 strain, Streptomyces fimicarius ATCC 21900 strain, Pseudomonas chlororaphis NBRC 3521 strain, Achromobacter denitrificans NARC 12669 strain, Achromobacter denitrificans ATCC 35699 strain, Raoultella planticola JCM 7251 strain and Raoultella terrigena JCM 1687 strain are preferred, and Pseudomonas chlororaphis NBRC 3521 strain, Achromobacter denitrificans NBRC 12669 strain and Achromobacter denitrificans ATCC 35699 strain are more preferred.

Herein, the description that D-lactic acid is produced "in high optical purity" denotes that D-lactic acid is produced in about 70%e.e. or higher in terms of enantiomer excess. D-lactic acid exists in the product preferably in about 80%e.e. or higher, more preferably in about 90°e.e. or higher, more preferably in about 95%e. e. or higher, more preferably in about 97%e.e. or higher, more preferably in 98%e.e. or higher, more preferably in 99%e.e. or higher, and further preferably in 99.9%e.e. or higher optical purity. The optical purity/enantiomer excess is determined according to a known method. For example, the method described in Japanese Unexamined Patent Publication (Kokai) No.2003-88392 can be used, but the determination method is not limited thereto. The selection of a bacterial strain which produces B-lactic acid in high optical purity is performed by culturing a candidate bacterial strain in a small scale using a test tube or flask, and measuring the optical purity of D-lactic acid, which is accumulated in a culture medium, by the above method.

The Pseudomonas auricularis NBRC 13334 strain, Pseudomonas, chlororaphis NBRC 3521 strain, Pseudomonas taetrolens NBRC 3460 strain, Achromobacter denitrificans NBRC 12669 strain, Devosia riboflavina NBRC 13584 strain, Frateuria aurantia NBRC 3247 strain, Paenibacillus validus NBRC 13635 strain, Rhizobium radiobacter NBRC 13532 strain, Serratia marcesceus NBRC 12648 strain, Streptomyces alboflavus NBRC 13196 strain, Streptomyces althioticus NBRC 15956 strain and Hafnia alvei NBRC 3731 strain are available from Biological Resource Center, Department of Biotechnology, Incorporated Administrative Agency National Institute of Technology and Evaluation, 2-5-8 Kazusakamatari, Kisarazu-shi, Chiba 292-0818 Japan. The Achromobacter denitrificans ATCC 35699 strain, Pseudomonas species ATCC 53617 strain, Streptomyces fimicarius ATCC 21900 strain, Escherichia coli ATCC 700926 strain and Brevibacterium butanicum ATCC 21196 strain are available from American Type Culture Collection, P.O. Box 1549, Manassas, VA 20108 URSA. The Pseudomonas azotoformans JCM 2777 strain, Arthrobacter nicotianae JCM 1333 strain, Arthrobacter protophormiae JCM 1973 strain, Pseudomonas fragi JCM 20552 strain, Raoultella planticola JCM 7251 strain and Raoultella terrigena JCM 1687 strain are available from Microbe Division / Japan Collection of Microorganisms, RIKEN BioResource Center, 2-1 Hirosawa, Wako, Saitama 351-Ort98, Japan. The Agrobacterium radiobacter FERM BP-3843 strain has been deposited under the Budapest Treaty since April 23, 1992, at International Patent Organism Depositary, National Institute of Advanced Industrial Science and Technology (AIST), AIST Tsukuba Central 6, 1 -1, Higashi l-chome Tsukuba-shi, Ibaraki-ken 305-8566, Japan.

The lactic acid-producing bacteria used for the present invention are specifically exemplified by the bacterial genera, bacterial species and bacterial strains described in the above 1 to 7 and 9 to 13. Among the lactic acid-producing bacteria, preferred bacteria are those that can produce lactic acid from glycerol in higher conversion rate. Herein, the "conversion rate" from glycerol to lactic acid denotes the ratio of lactic acid to be produced (mol) to glycerol to be consumed (mol) in terms of amount. The conversion rate is calculated by the following equation. Conversion rate (%) = [Amount of lactic acid to be produced (mol)/Amount of glycerol to be consumed (mol)] x 100 The description that lactic acid is produced "in higher conversion rate" denotes that lactic acid is produced from glycerol in a conversion rate of about 10% (mol/mol) or higher. In general, the higher the conversion rate, the better it is. For example, lactic acid is produced in a conversion rate of about 20% (mol/mol) or higher, more preferably in a conversion rate of about 40% (mol/mol) or higher.

Examples of such a bacterium include the Proteus vulgaris NARC 3851 strain, Nocardioides simplex NBRC 12069 strain, Nocardia uniformis NBRC 13702 strain, Bacillus sphaericus NBRC 3341 strain, Bacillus badius ATCC 14574 strain, Arthrobacter aurescens NBRC 12136 strain, Arthrobacter citreus NBRC 12957 strain, Streptomyces carnosus NBRC 13025 strain and Streptomyces cellulosae NBRC 3713 strain, but it is not limited thereto. Preferred bacteria are the Arthrobacter aurescens NBRC 12136 strain, Arthrobacter citreus NBRC 12957 strain, Bacillus badius ATCC I4574 strain, Nocardia uniformis NBRC 13702 strain, Proteus vulgaris NBRC 3851 strain and Streptomyces cellulosae NBRC 3713 strain. Streptomyces cellulosae NBRC 3713 strain is more preferred.

The Arthrobacter aurescens NBRC 12136 strain, Arthrobacter citreus NBRC 12957 strain. Bacillus sphaericus NBRC 3341 strain, Nocardia uniformis NBRC 13702 strain, Nocardioides simplex NBRC 12069 strain, Proteus vulgaris NBRC 3851 strain, Streptomyces carnosus NBRC 13025 strain and Streptomyces cellulosae NBRC 3713 strain are available from Biological Resource Center, Department of Biotechnology, Incorporated Administrative Agency National Institute of Technology and Evaluation, 2-5-8 Kazusakamatari, Kisaraxu-shi, Chiba 292-0818 Japan. The Bacillus badius ATCC 14574 strain is available from American Type Culture Collection, P.O.Box 1549, Manassas, VA 20108 URSA.

The lactic acid-producing bacteria or D-lactic acid-producing bacteria used for the present invention may not only be their wild-type strains, but also be any given naturally-occurring or artificial mutants, including those obtained by treatment with X-ray irradiation, ultraviolet-ray irradiation or a chemical mutagen such as N-methyl-N'-nitro-N-nitrosoguanidine, or recombinants obtained through genetic engineering techniques such as cell fusion and gene recombination. A host of the recombinant can belong to any bacterial genera as long as it is a transformable microorganism. However, it is preferred that the host belongs to the same bacterial genus as a parent strain that a targeted gene is originated from. It is desirable to select a D-lactic acid-producing bacterium with improved conversion capacity from glycerol into lactic acid or D-.lactic acid, or that with further improved optical purity of D-lactic acid.

A culture medium containing glycerol may be a medium obtained by adding pure glycerol or a glycerol-containing mixture. Components other than glycerol in the glycerol-containing mixture or their amount are preferably those which do not have harmful effects on the lactic acid-producing bacteria or D-lactic acid-producing bacteria used for the present invention. Origin of the glycerol-containing mixture is not particularly limited, but using Bio Diesel waste is preferable for effective use of resources.

One of a method for producing Bio Diesel Fuel is to produce Fatty Acid Methyl Ester (FAME) by alcoholysis of triglyceride using an alkali catalyst. In this method, waste fluid containing glycerol is produced as a by-product (called Bio Diesel waste). This waste fluid is usually contaminated with a catalyst, unconverted fatty acids (they differ depending on used oil) and the like. For example, the composition of a Bio Diesel waste used after-mentioned Examples is glycerol: 51%, methanol: 11%, potassium hydroxide: 8%, water: 4%, others such as glyceride: 26%. Bio Diesel waste fluids having arbitrary composition, such as glycerol: 65%, potassium/sodium salt: 4 to 5%, methanol: 1%, water: 28% described in S. Papanikolaou et al., Bioresource Technology (2002) 82: 43-49, and glycerol: 65%, sodium salt: 5% or less described in M. Gonzalez-Pajuelo et al. , J IndMicrobiol Biotechnol (2004) 31: 442-446, can also be used.

When a Bio Diesel waste is added to a medium in a method of the present invention, it is possible to produce lactic acid or B-lactic acid in the same or higher level of yield and conversion efficiency as in the case of adding pure glycerol.

The medium used for the method of the present invention may be any kind as long as it contains common components necessary for culture of bacteria, and is not limited to a particular medium. In the present invention, it is possible to obtain D-lactic acid in high optical purity, or to obtain lactic acid in high conversion rate, even in a medium with plain composition containing carbon sources, nitrogen sources and inorganic salts.

The medium used for the method of the present invention contains glycerol as a carbon source. The concentration of glycerol contained in the medium can be properly selected within a range which exerts no harmful effect on growth of bacteria, and production of lactic acid or D-lactic acid. However, it is usually from about 0.1 to 500 g/L, and preferably from about 1 to 300 g/L. When the Bio Diesel waste is used as a glycerol source, it is allowed to dilute the waste liquid, or to add glycerol thereto, until the amount of glycerol in the medium falls within the above range, depending on the concentration of glycerol contained in the waste.

The medium may contain substances other than glycerol as carbon sources, but the amount thereof should be limited to the extent which does not interfere production of lactic acid or D-lactic acid from glycerol. The carbon sources used for the present invention are exemplified by glucose, fructose, starch, lactose, arabinose, xylose, dextrin, molasses and malt extract, but are not limited thereto. The amount of other carbon sources is preferably about 10% by weight or less of glycerol, and more preferably about 1% by weight or less. It is most preferable that the medium contains glycerol as a single carbon source.

Examples of a nitrogen source include inorganic nitrogen compounds such as ammonia, ammonium sulfate, ammonium chloride and ammonium nitrate, urea, and the like. It is also allowed to use organic nitrogen sources such as gluten flour, cottonseed flour, soybean flour, corn steep liquor, dried yeast, yeast extract, peptone, meat extract and casamino acid.

It is advantageous to use carbon sources and nitrogen sources in combination. Since the sources of low purity, containing a trace amount of growth factors and a great deal of inorganic nutrients, are also suitable for the use, there is no need to use them in a pure form.

Upon request, it is allowed to use inorganic salts, such as potassium phosphate monobasic, potassium phosphate dibasic, sodium chloride, magnesium sulfate, manganese sulfate, calcium carbonate, calcium chloride, sodium iodide, potassium iodide, and cobalt chloride. It is also allowed to add defoaming agents, such as liquid paraffin, higher alcohol, vegetable oil, mineral oil and silicon, as needed, particularly when the medium foams markedly.

Other components such as various vitamins may be added to the medium, as needed.

The nitrogen sources, inorganic salts and other components are known by those skilled in the art.

In the present invention, it is allowed to culture lactic acid-producing bacteria or D-lactic acid-producing bacteria under an anaerobic condition, but preferably it is performed under an aerobic condition. The aerobic condition denotes culture in the presence of molecular oxygen. Ventilation, stirring and shaking can be performed for supplying oxygen. It is available to use any common devices for culture of microorganism. In the case of culturing under an aerobic condition, the method of the present invention allows to culture bacteria, and to produce lactic acid or D-lactic acid, by a simple manner without using any devices necessary for bringing about an anaerobic condition.

Culture of bacteria under an anaerobic condition can be performed by introducing carbon dioxide or inert gas (nitrogen argon, etc.), or without ventilation.

It is preferred for mass production of lactic acid or D-lactic acid to be performed under a submerged culture condition. When bacteria are propagated in a large tank, it is preferred to inoculate bacteria in a vegetative period into a production tank, so as to avoid delay in propagation in a lactic acid or D-lactic acid producing process. That is, it is preferred that bacteria are first inoculated to a relatively small amount of medium, and cultured to produce seed bacteria in a vegetative period, and then the seed bacteria is transferred into the large tank in a sterile manner.

Stirring and ventilation of the culture solution can be performed in various manners. Stirring can be performed using a propeller or a mechanical stirring device similar to a propeller, rotation or shake of a fermenter, or a pumping device. Ventilation can be performed by allowing sterilized air to pass through in the culture solution. In doing so, the ventilation operation may provide stirring effect as well.

In the case of culture in a submerged medium, a culture method such as batch culture, fed batch culture and continuous culture can be properly selected and used.

The culture conditions are dis4retional as long as they are suitable for culture of lactic acid-producing bacteria or D-lactic acid-producing bacteria used for the present invention. For example, the culture temperature is from about 4 to 40°C, preferably from about 20 to 37 °C. The pH of the medium is from about 5 to 9, and preferably from about 6 to 8. When the pH of the medium-declines along with production of lactic acid or D-lactic acid, it is adjusted to be fallen within the above range, by adding alkali such as an aqueous ammonia solution, calcium carbonate, sodium hydroxide and potassium hydroxide to the culture system, as needed.

The composition of the medium and other culture conditions are appropriately adjustable by those skilled in the art. It will also be considered to adjust the conditions, in order to further enhance yield and optical purity of lactic acid or D-lactic acid.

The bacteria used for the method of the present invention may take a bacterial cell, processed bacterial cell or immobilized product thereof. Herein, the processed bacterial cell denotes a disrupted bacterial cell or an enzyme extracted from cultured substances (include a bacterial cell and culture supernatant). Examples of the processed bacterial cell include that obtained by treating a cultured bacterial cell with an organic acid (such as acetone and ethanol), freeze dry treatment or alkali treatment, that obtained by physically or enzymatically disrupting a bacterial cell, or a crude enzyme separated or extracted therefrom. Specifically, cultured bacteria are subjected to a centrifugal treatment, and the cells to be collected are disrupted by a physical milling method such as an ultrasonic, Dyno-mill and French press treatment, or a chemical disrupting method using a surfactant or a lytic enzyme such as lyzozyme. The resultant solution is subjected to centrifuge or membrane filtration to remove insoluble materials, and the resultant cell-free extract is subjected to a separation/purification method, such as cation exchange chromatography, anion exchange chromatography, hydrophobic chromatography, gel filtration chromatography and metal chelate chromatography, to fractionate and purify the enzyme.

Examples of a carrier used for the chromatography include insoluble polymer carriers such as cellulose, dextrin and agarose introduced with a carboxymethyl (CM) group, diethylaminoethyl (DEAE) group, phenyl group or butyl group. It is also allowed to use a commercially available carrier-packed column. Disruption of the bacterial cell and extraction of the enzyme can be performed by a known method by those skilled in the art, as well as the above method.

Examples of contacting the bacterial cell, processed bacteria cell or immobilized thereof to glycerol are given below.

The method for producing lactic acid or D-lactic acid from glycerol using a bacterial cell or processed bacterial cell is exemplified by a method that the bacterial cell is suspended and reacted in a glycerol-containing substrate solution. The bacterial cell can be prepared by culturing lactic acid-producing bacteria or D-lactic acid-producing bacteria, followed by centrifuge thereof. It is preferred that the concentration of glycerol in the substrate solution is approx. from 0.01 to 50% by weight. The reaction temperature is usually from about 4 to 40°C, and preferably from about 20 to 37°C. The pH of the reaction solution is usually from about 5 to 9, and preferably from 6 to 8. When the pH of the medium declines along with production of lactic acid or D-lactic acid, it is adjusted to be fallen within the above range, by adding alkali such as an aqueous ammonia solution, calcium carbonate, sodium hydroxide and potassium hydroxide to the culture system, as needed.

The method for producing lactic acid or D-lactic acid from glycerol using an immobilized bacterial cell or processed bacterial cell is exemplified by a method that the immobilized bacterial cell or processed bacterial cell is filled in a column, and a glycerol-containing substrate solution is allowed to pass it through. The bacterial cell or processed bacterial cell is obtained by culturing the lactic acid-producing bacteria or D-lactic acid-producing bacteria, followed by centrifuge thereof. The method for im_rnobi2izing the bacterial cell is exemplified by a comprehensive immobilization means using a gel, and immobilization means by supporting an ion exchange material. Examples of the gel to be used include carrageenan, agar, mannan, PVAand polyacrylamide gels. The proper particle size of the gel is from about 1 to 10 mum in diameter, although the size varies depending on a kind of gel. Examples of the ion exchange material include a cellulose-based material, styrenedivinylbenzene-based material and phenolformalin-based ion exchange material. It is preferred that the concentration of glycerol in the substrate solution is from about 0.01 to 50% by weight. It is also allowed to add a SH compound such as mercaptoethanol, cysteine and glutathione, reducing agent such as sulfide, and enzyme activator such as a magnesium ion and manganese ion. The velocity of the solution passing through varies depending on the column size and amount of the immobilized substance. It is proper that the space velocity (ml/ml resin·hr) is from 0.05 to 10, as an index of velocity for treating a solution.

Separation and purification of lactic acid or D-lactic acid are performed in accordance with a conventional known method. For example, lactic acid or D-lactic acid is separated and purified by a methods such as acidification of a cultured material and direct distillation thereof, formation of lactide of lactic acid and distillation thereof, esterification of lactic acid by adding alcohol and a catalyst and distillation thereof, extraction of lactic acid in an organic solvent, adsorbing lactic acid to an ion exchange resin and separation thereof using an ion exchange column which enables elusion separation, formation of a metal salt with a calcium ion or the like and isolation thereof, and concentration separation of lactic acid by electrodialysis. Specifically, separation and purification of lactic acid may be performed in accordance with Examples described in Japanese Unexamined Patent Publication (Kokai) No.2003-88392, for example.

According to the method of the present invention, D-lactic acid is prepared from glycerol (Bio Diesel waste) using a microorganism in high optical purity by a simple manner.

Polylactic acid is a biodegradable polymer (or called bioplastic), which is synthesized from lactic acid originated from plant material. Poly-L-lactic acid synthesized by polymerization of L-lactic acid attracts attention as an ecological material, but the heat resistance is inferior to conventional plastics. However, it is known that by using a mixture of poly-L-lactic acid and pcly-B-3actic acid (also called stereocomp3ex-type polylactic acid), heat resistance of the resultant biodegradable polymer improves.

According to the method of the present invention, lactic acid is prepared from glycerol (Bio Diesel waste) using a microorganism in high conversion rate by a simple manner.

Lactic acid is utilized for diverse usages, such as a food additive in food business, a pH adjuster or a bioplastic material for industrial use, and for infusion and intestinal disinfection in medical use.

Thus, the present invention provides a method of producing useful materials from glycerol contained in a Bio Diesel waste, which used to be discarded.

The present invention is further illustrated by the following examples. It is to be understood that the present invention is not limited to the examples, and various variations can be made within a range of the present invention.

### EXAMPLES

### Example 1

Achromobacter denitrificans NARC 12669 strain was spread on an agar medium A, being a medium for plate culture, (composition: 3 g of potassium phosphate monobasic, 6 g of sodium phosphate dibasic, 0.5 g or sodium chloride, 1 g of ammonium chloride, 492 mg of magnesium sulfate heptahydrate, 147 mg of calcium chloride dihydrate, 100 mg of yeast extract, 10 got glycerol, 20 g of agar and 1 L of distilled water (final pH 7.4)), and allowed to stand at 30°C for 4 days. The bacterial strain grown on the above plate was inoculated with a platinum loop in 3 mL of a medium B, being a medium for test tube culture (composition: the same composition as the agar medium A, except for containing no calcium chloride, yeast extract and agar), and subjected to shaking culture (pre-culture) at 30°C at 200 rpm for 24 hours. The bacterial strain culture solution of 30 µL grown above was transferred to 3 mL of a medium C, being a medium for test tube culture (composition: the same composition as the above medium B for test tube culture, except for containing 19.6 g of a glycerol fraction (glycerol: 51%, methanol: 11%, potassium hydroxide: 8%, water: 4%, and others including glyceride: 26%) which was by-produced upon production of the Bio Diesel Fuel, instead of 10 g of glycerol), and subjected to shaking culture (main culture) at 30°C at 200 rpm. Four (4) days after initiation of the reaction, 3. 9 g of glycerol was consumed per litter, and 3.1 g of lactic acid was accumulated. The optical purity of D-lactic acid was 99.9%e.e. or higher.

### Example 2

Achromobacter denitrificans NBRC 12669 strain was spread on an agar medium A, being a medium for plate culture, (composition: 3 g of potassium phosphate monobasic, 6 g of sodium phosphate dibasic, 0.5 g or sodium chloride, 1 g of ammonium chloride, 492 mg of magnesium sulfate heptahydrate, 147 mg of calcium chloride dihydrate, 100 mg of yeast extract, 10 g of glycerol, 20 g of agar and 1L of distilled water (final pH 7.4)), and allowed to stand at 30°C for 4 days. The bacterial strain grown on the above plate was inoculated with a platinum loop in 3 mL of a medium D, being a medium for test tube culture (composition: the same composition as the agar medium A, except for containing no agar), and subjected to shaking culture (pre-culture) at 30°C at 200 rpm for 24 hours. The bacterial strain culture solution of 30 µL grown above was transferred to 3 mL of a medium E, being a medium for test tube culture, (composition: the same composition as the above medium D for test tube culture, except for containing 19.6 g of a glycerol fraction (glycerol: 51%, methanol: 11%, potassium: hydroxide: 8%, water: 4%, and others including glyceride: 26%) which was by-produced upon production of the Bio Diesel Fuel, instead of 10 g of glycerol), and subjected to shaking culture (main culture) at 30°C at 200 rpm. Four (4) days after initiation of the reaction, 9.4 g of glycerol was consumed per litter, and 3.9g of lactic acid was accumulated. The optical purity of D-lactic acid was 99.9%e.e. or higher.

### Example 3

Pseudomonas chlororaphis NBRC 3521 strain was reacted in the same manner as in Example 1. As a result, 7.5 g of glycerol was consumed per litter, and 0.9 g of lactic acid was accumulated. The optical purity of D-lactic acid was 99.9%e.e or higher.

### Example 4

Pseudomonas azotoformans JCM 2777 strain was reacted in the same manner as in Example 1. As a result, 3.3 g of glycerol was consumed per litter, and 0. 4 g of lactic acid was accumulated. The optical purity of D-lactic acid was 99.9%e.e or higher.

### Example 5

Pseudomonas auricularis NBRC 13334 strain was reacted in the same manner as in Example 1. As a result, 3.5 g of glycerol was consumed per litter, and 0.4 g of lactic acid was accumulated. The optical purity of D-lactic acid was 99.9%e.e or higher.

### Example 6

Agrobacterium radiobacter FERM BP-3843 strain was reacted in the same manner as in Example 1. As a result, 0'.4 g of glycerol was consumed per litter, and 0.1 g of lactic acid was accumulated. The optical purity of D-lactic acid was 99.9%e.e or higher.

### Example 7

Achromobacter denitrificans NBRC 12669 strain was spread on an agar medium A, being a medium for plate culture, (composition: 3g of potassium phosphate monobasic, 6 g of sodium phosphate dibasic, 0.5 g or sodium chloride, 1 g of ammonium chloride, 492 mg of magnesium sulfate heptahydrate, 147 mg of calcium chloride dihydrate, 100 mg of yeast extract, 10 g of glycerol, 20 g of agar and 1L of distilled water (final pH 7.4)), and allowed to stand at 30°C for 4 days. The bacterial strain grown on the above plate was inoculated with a platinum loop in 3 mL of a medium B, being a medium for test tube culture (composition: the same composition as the agar medium A, except for containing no calcium chloride, yeast extract and agar), and subjected to shaking culture (pre-calture) at 30°C at 200 rpm for 24 hours. The bacterial strain culture solution of 30 µL grown above was transferred to 3 mL of the medium B, and subjected to shaking culture (main culture) at 30°C at 200 rpm. Four (4) days after initiation of the reaction, 1.7 g of glycerol was consumed per litter, and 0.3 g of lactic acid was accumulated. The optical purity of D-lactic acid was 99. 9%e. e. or higher.

### Example 8

Paenibacillus validus NBRC 13635 strain was reacted in the same manner as in Example 7. As a result, 0.3 g of glycerol was consumed per litter, and 0.2 g of lactic acid was accumulated. The optical purity of D-lactic acid was 94.0%e.e.

### Example 9

Pseudomonas chlororaphis NBRC 3521 strain was reacted in the same manner as in Example 7. As a result, 7.7 g of glycerol was consumed per litter, and 2.1 g of lactic acid was accumulated. The optical purity of D-lactic acid was 99.9%e.e. or higher.

### Example 10

Agrobacterium radiobacter FERM BP-3843 strain was reacted in the same manner as in Example 7. As a result, 1.7 g of glycerol was consumed per litter, and 0.3 g of lactic acid was accumulated. The optical purity of D-lactic acid was 98.3%e.e.

### Example 11

Serratia marcesceus NBRC 12648 strain was reacted in the same manner as in Example 7. As a result, 9.4 g of glycerol was consumed per litter, and 1.4 g of lactic acid was accumulated. The optical purity of D-lactic acid was 99.2%e.e.

### Example 12

Achromobacter denitrificans NBRC 12669 strain was spread on an agar medium A, being a medium for plate culture, (composition: 3 g of potassium phosphate monobasic, 6 g of sodium phosphate dibasic, 0.5 g or sodium chloride, 1 g of ammonium chloride, 492 mg of magnesium sulfate heptahydrate, 147 mg of calcium chloride dihydrate, 100 mg of yeast extract, 10 g of glycerol, 20 g of agar and 1L of distilled water (final pH 7.4)), and allowed to stand at 30°C for 4 days. The bacterial strain grown on the above plate was inoculated with a platinum loop in 3 mL of a medium D, being a medium for test tube culture (composition: the same composition as the agar medium A, except for containing no agar), and subjected to shaking culture (pre-culture) at 30°C at 200 rpm for 24 hours. The bacterial strain culture solution of 30 µL grown above was transferred to 3 mL of the medium D, being a medium for test tube culture, and subjected to shaking culture (main culture) at 30°C at 200 rpm. Four (4) days after initiation of the reaction, 3.4 g of glycerol was consumed per litter, and 1.2 g of lactic acid was accumulated. The optical purity of D-lactic acid was 99.9% e. e. or higher.

### Example 13

Paenibacillus validus NBRC 13635 strain was reacted in the same manner as in Example 12, except for changing the pre-culture period from 24 hours to 6 days, and the main culture period from 4 days to 6 days. As a result, 1.1 g of glycerol was consumed per litter, and 0.2 g of lactic acid was accumulated. The optical purity of D-lactic acid was 94.0%e.e.

### Example 14

Frateuria aurantia NBRC 3247 strain was reacted in the same manner as in Example 12, except for changing the pre-culture period from 24 hours to 6 days, and the main culture period from 4 days to 6 days. As a result, 2.4 g of glycerol was consumed per litter, and 0.3 g of lactic acid was accumulated. The optical purity of D-lactic acid was 99.9%e.e. or higher.

### Example 15

Devosia, riboflavina NBRC 13584 strain was reacted in the same manner as in Example 12, except for changing the pre-culture period from 24 hours to 6 days, and the main culture period from 4 days to 6 days. As a result, 0.9 g of glycerol was consumed per litter, and 0.1 g of lactic acid was accumulated. The optical purity of D-lactic acid was 95.5%e.e.

### Example 16

Streptomyces fimicarius ATCC 21900 strain was reacted in the same manner as in Example 12. As a result, 7.6 g of glycerol was consumed per litter, and 1.0 g of lactic acid was accumulated. The optical purity of D-lactic acid was 95.5%e.e.

### Example 17

Escherichia coli ATCC 700926 strain was reacted in the same manner as in Example 7. As a result, 9.9 g of glycerol was consumed per litter, and 1.0 g of lactic acid was accumulated. The optical purity of D-lactic acid was 99.9%e.e. or higher.

### Example 18

Brevibacterium butanicum ATCC 21196 strain was reacted in the same manner as in Example 2. As a result, 2.8 g of glycerol was consumed per litter, and 0.3 g of lactic acid was accumulated. The optical purity of D-lactic acid was 99.9%e.e. or higher.

### Example 19

Pseudomonas taetrolens NBRC 3460 strain was reacted in the same manner as in Example 1, except for changing the pre-culture period from 24 hours to 6 days, and the main culture period from 4 days to 6 days. 0.7 g of glycerol was consumed per litter, and 0.3 g of lactic acid was accumulated. The optical purity of D-lactic acid was 99.9%e.e. or higher.

### Example 20

Streptomyces alboflavus NBRC 13196 strain was reacted in the same manner as in Example 12, except for changing the pre-culture period from 24 hours to 6 days, and the main culture period from 4 days to 6 days. As a result, 9. 9 g ot glycerol was consumed per litter, and 0.2 g of lactic acid was accumulated. The optical purity of D-lactic acid was 95.9%e.e.

### Example 21

Streptomyces althioticus NBRC 15956 strain was reacted in the same manner as in Example 12, except for changing the pre-culture period from 24 hours to 6 days, and the main culture period from 4 days to 6 days. As a result, 2.2 g of glycerol was consumed per litter, and 0.1 g of lactic acid was accumulated. The optical purity of D-lactic acid was 96.3%e.e.

### Example 22

Hafnia alvei, NBRC 3731 strain was reacted in the same manner as in Example 2. As a result, 10.5 g of glycerol was consumed per litter, and 0.1 g of lactic acid was accumulated. The optical purity of D-lactic acid was 99.9%e.e. or higher.

### Example 23

Arthrobacter nicotianae JCM 1333 strain was reacted in the same manner as in Example 12. As a result, 7.1 g of glycerol was consumed per litter, and 0.1 g of lactic acid was accumulated. The optical purity of D-lactic acid was 99.9%e.e. or higher.

### Example 24

Arthrobacter protophormiae JCM 1973 strain was reacted in the same manner as in Example 12. As a result, 3.7 g of glycerol was consumed per litter, and 0. 1 g of lactic acid was accumulated. The optical purity of D-lactic acid was 99.9%e,e, or higher.

### Example 25

Pseudomonas fragi JCM 20552 strain was reacted in the same manner as in Example 2. As a result, 4.5 g of glycerol was consumed per litter, and 0.3 g of lactic acid was accumulated. The optical purity of D-lactic acid was 99.9%e.e. or higher.

### Example 26

Raoultella planticola JCM 7251 strain was reacted in the same manner as in Example 7. As a result, 9.9 g of glycerol was consumed per litter, and 0. 9 g of lactic acid was accumulated. The optical purity of D-lactic acid was 97.2%e.e.

### Example 27

Raoultella terrigena JCM 1687 strain was reacted in the same manner as in Example 1. As a result, 9.2 g of glycerol was consumed per litter, and 0.6 g of lactic acid was accumulated. The optical purity of D-lactic acid was 99.9%e.e. or higher.

### Example 28

Rhizobium radiobacter NBRC 13532 strain was reacted in the same manner as in Example 2. As a result, 2.0 g of glycerol was consumed per litter, and 0.2 g of lactic acid was accumulated. The optical purity of D-lactic acid was 99.9%e.e. or higher.

### Example 30

Escherichia coli ATCC 700926 strain was spread on an agar medium H, being a medium for plate culture, (composition: 3 g of potassium phosphate monobasic, 6 g of sodium phosphate dibasic, 0.5 g of sodium chloride, 1 g of ammonium chloride, 250 mg of magnesium sulfate heptahydrate, 20 g of glycerol, 20 g of agar and 1L of distilled water (final pH 7.4)), and allowed to stand at 30°C for 4 days. The bacterial strain grown on the above plate was inoculated with a platinum loop in 3 mL of a medium F, being a medium for test tube culture (composition: the same composition as the agar medium H, except for containing no agar and containing 10 g or glycerol instead of 20 g), and subjected to shaking culture at 30°C at 200 rpm for 24 hours. The bacterial strain culture solution of 30 µL grown above was transferred to 3 mL of the medium F, being a medium for test tube culture, and subjected to shaking culture at 30°C at 200 rpm for 24 hours. Five (5) days after initiation of the reaction, 0.72g/L of lactic acid was accumulated. The optical purity of D-lactic acid was 99.9%e.e. or higher.

### Example 31

Escherichia coli ATCC 700926 strain was spread on an agar medium H, being a medium for plate culture, (composition: 3 g of potassium phosphate monobasic, 6 g of sodium phosphate dibasic, 0.5 g or sodium chloride, 1 g of ammonium chloride, 250 mg of magnesium sulfate heptahydrate, 20 g of glycerol, 20 g of agar and 1L of distilled water (final pH 7.4)), and allowed to stand at 30°C for 4 days. The bacterial strain grown on the above plate was inoculated with a platinum loop in 3 mL of a medium F, being a medium for test tube culture (composition: the same composition as the agar medium H, except for containing no agar and containing 10 g or glycerol instead of 20 g), and subjected to shaking culture at 30°C at 200 rpm for 24 hours. The bacterial strain culture solution of 30 µL grown above was transferred to 3 mL of a medium G, being a medium for test tube culture, (composition: the same composition as the above test tube culture medium F, except for containing 98.0 g of a glycerol fraction (glycerol 51%, methanol 11%, potassium hydroxide 8%, water 4%, and others including glyceride 26%) which was by-produced upon production of the Bio Diesel Fuel, instead of 10 g of glycerol) and subjected to shaking culture at 30°C at 200 rpm for 24 hours. Five (5) days after initiation of the reaction, 0.24g/L of lactic acid was accumulated. The optical purity of D-lactic acid was 99.9%e.e. or higher.

### Example 32

Pseudomonas species ATCC 53617 strain was reacted in the same manner as in Example 7. As a result, 1. 8 g of glycerol was consumed per litter, and 0.2 g of lactic acid was accumulated. The optical purity of D-lactic acid was 99.9%e.e. or higher.

### Example 33

Achromobacter denitrificans ATCC 35699 strain was reacted in the same manner as in Example 7, except for changing the main culture period from 4 days to 6 days. As a result, 8.9 g of glycerol was consumed per litter, and 2.7 g of lactic acid was accumulated. The optical purity of D-lactic acid was 99.9%e.e. or higher.

### Example 34

Arthrobacter citrous NBRC 12957 strain was spread on an agar medium A, being a medium for plate culture, (composition: 3 g of potassium phosphate monobasic, 6 g of sodium phosphate dibasic, 0.5g or sodium chloride, 1 g of ammonium chloride, 492 mg of magnesium sulfate heptahydrate, 147 mg of calcium chloride dihydrate, 100 mg of yeast extract, 10 g of glycerol, 20 g of agar and 1L of distilled water (final pH 7.4)), and allowed to stand at 30°C for 4 days. The bacterial strain grown on the above plate was inoculated with a platinum loop in 3 mL of a medium D, being a medium for test tube culture (composition: the same composition as the agar medium A, except for containing no agar), and subjected to shaking culture (pre-culture) at 30°C at 200 rpm for 24 hours. The bacterial strain culture solution of 30 µL grown above was transferred to 3 mL of the medium D, being a medium for test tube culture, and subjected to shaking culture (main culture) at 30°C at 200 rpm. 4 days after initiation of the reaction, 2.4 g of glycerol was consumed per litter, 0.5 g of lactic acid was accumulated, and the conversion rate was 21% (mol/mol).

### Example 35

Arthrobacter aurescens NBRC 12136 strain was reacted in the same manner as in Example 34. As a result, 2.5 g of glycerol was consumed per litter, 0.5 g of lactic acid was accumulated, and the conversion rate was 20% (mol/mol).

### Example 36

Bacillus badius ATCC 14574 strain was reacted in the same manner as in Example 34. As a result, 2.7 g of glycerol was consumed per litter, 0.5 g of lactic acid was accumulated, and the conversion rate was 19% (mol/mol).

### Example 37

Bacillus sphaericus NBRC 3341 strain was reacted in the same manner as in Example 34. A,s a result, 1. 9 g of glycerol was consumed per litter, 0.3 g of lactic acid was accumulated, and the conversion rate was 16% (mol/mol).

### Example 38

Nocardia uniformis NBRC 13702 strain was reacted in the same manner as in Example 34. As a result, 3.7 g of glycerol was consumed per litter, 0.5 g of lactic acid was accumulated, and the conversion rate was 14% (mol/mol).

### Example 39

Nocardia simplex NBRC 12069 strain was reacted in the same manner as in Example 34, except for changing the pre-culture period from 24 hours to 6 days, and the main culture period from 4 days to 6 days. As a result, 2.2 g of glycerol was consumed per litter, 0.3 g of lactic acid was accumulated, and the conversion rate was 14% (mol/mol).

### Example 40

Proteus vulgaris NBRC 3851 strain was reacted in the same manner as in Example 34, except for changing the pre-culture period from 24 hours to 6 days, and the main culture period from 4 days to 6 days. As a result, 5.3 g of glycerol was consumed per litter, 0. 6 g of lactic acid was accumulated, and the conversion rate was 12% (mol/mol).

### Example 41

Streptomyces carnosus NBRC 13025 strain was reacted in the same manner as in Example 34, except for changing the pre-culture period from 24 hours to 6 days, and the main culture period from 4 days to 6 days. As a result, 1.8 g of glycerol was consumed per litter, 0.2 g of lactic acid was accumulated, and the conversion rate was 11% (mol/mol).

### Example 42

Streptomyces cellulosae NBRC 3713 strain was reacted in the same manner as in Example 34. As a result, 12.4 g of glycerol was consumed per litter, 2.1 g of lactic acid was accumulated, and the conversion rate was 17% (mol/mol).

### Example 43

Streptomyces cellulosae NBRC 3713 strain was spread on an agar medium A, being a medium for plate culture, (composition: 3 g of potassium phosphate monobasic, 6 g of sodium phosphate dibasic, 0.5 g or sodium chloride, 1 g of ammonium chloride, 492 mg of magnesium sulfate heptahydrate, 147 mg of calcium chloride dihydrate, 100 mg of yeast extract, 10 g of glycerol, 20 g of agar and 1 L of distilled water (final pH 7.4)), and allowed to stand at 30°C for 4 days. The bacterial strain grown on the above plate was inoculated with a platinum loop in 3 mL of a medium B, being a medium for test tube culture (composition: the same composition as the agar medium A, except for containing no calcium chloride, yeast extract and agar), and subjected to shaking culture (pre-culture) at 30°C at 200 rpm for 24 hours. The bacterial strain culture solution of 30 µL grown above was transferred to 3 mL of the medium B, being a medium for test tube culture, and subjected to shaking culture (main culture) at 30°C at 200 rpm. Four (4) days after initiation of the reaction, 7.5 g of glycerol was consumed per litter, 1.0 g of lactic acid was accumulated, and the conversion rate was 14% (mol/mol).

### INDUSTRIAL APPLICABILITY

According to the present invention, D-lactic acid is produced from glycerol (Bio Diesel waste), using a microorganism, in high conversion rate, in high yield, and by a simple manner. In addition, D-lactic acid is prepared in high optical purity by a simple manner. D-lactic acid is used as a bioplastic material. According to the present invention, lactic acid is also produced from glycerol (Bio Diesel waste), using a microorganism, in high conversion rate, and by a simple manner. Lactic acid is used for diverse usages, such as a food additive in food business, a pH adjuster or a bioplastic material for industrial use, and for infusion or intestinal disinfection in medical use. Thus, the present invention serves for producing useful substances from a waste material.

The present invention also relates to the following items:
1. A method for producing lactic acid from glycerol, which comprises culturing any one of the following bacteria in a culture medium containing glycerol or contacting cells of the bacterium, processed cells of the bacterium or immobilized product thereof with glycerol:
   (a) a bacterium belonging to a genus selected from the bacterial genus group consisting of Agrobacterium, Pseudomonas, Achromobacter, Devosia, Frateuria, Paenibacillus, Brevibacterium, Hafnia, Raoultella, Rhizobium and Serratia, and being capable of producing D-lactic acid from glycerol in about 70%e.e. or higher optical purity;
   (b) a bacterium belonging to a species selected from the bacterial species group consisting of Arthrobacter nicotianae, Arthrobacter protophormiae, Streptomyces fimicarius, Streptomyces alboflavus and Streptomyces althioticus, and being capable of producing D-lactic acid from glycerol in about 70%e.e. or higher optical purity;
   (c) a bacterium of Escherichia coli ATCC 700926 strain;
   (d) a bacterium belonging to a genus selected from the bacterial genus group consisting of Nocardioides and Proteus, and being capable of producing lactic acid from glycerol; and
   (e) a bacterium belonging to a species selected from the bacterial species group consisting of Arthrobacter aurescens, Arthrobacter citreus. Bacillus badius, Bacillus sphaericus, Nocardia uniformis, Streptomyces carnosus and Streptomyces cellulosae, and being capable of producing lactic acid from glycerol.
2. A method for producing D-lactic acid from glycerol, which comprises:
   culturing in a culture medium containing glycerol, a bacterium belonging to a genus selected from the bacterial genus group consisting of Agrobacterium, Pseudomonas, Achromobacter, Devosia, Frateuria, Paenibacillus, Brevibacterium, Hafnia, Raoultella, Rhizobium and Serratia, and being capable of producing D-lactic acid from glycerol in about 70%e.e. or higher optical purity; or
   contacting cells of the bacterium, processed cells of the bacterium or immobilized product thereof with glycerol.
3. The method according to item 2, wherein a bacterium belonging to a genus selected from the bacterial genus group consisting of Agrobacterium, Pseudomonas, Achromobacter, Devosia, Frateuria, Paenibacillus, Brevibacterium, Hafnia, Raoultella, Rhizobium and Serrtia is a bacterium of Agrobacterium radiobacter, Pseudomonas auricularis, Pseudomonas azotoformans, Pseudomonas chlororaphis, Pseudomonas taetrolens, Pseudomonas fragi, Pseudomonas sp., Achromobacter denitrificans, Devosia riboflavina, Frateuria aurantia, Paenibacillus validus, Brevibacterium butanicum, Hafnia alvei, Raoultella planticola, Raoultella terrigera, Rhizobium radiobacter or Serratia marcesceus.
4. The method according to item 3, wherein a bacterium belonging to a genus selected from the bacterial genus group consisting of Agrobacterium, Pseudomonas, Achromobacter, Devosia, Frateuria, Paenibacillus, Brevibacterium, Hafnia, Raoultella, Rhizobium and Serratia is a bacterium of Agrobacterium radiobacter FERM BP-3843 strain, Pseudomonas auricularis NBRC 13334 strain, Pseudomonas azotoformans JCM 2777 strain, Pseudomonas chlororaphis NBRC 3521 strain, Pseudomonas taetrolens NBRC 3460 strain, Pseudomonas fragi JCM 20552 strain, Pseudomonas species ATCC 53617 strain, Achromobacter denitrificans NBRC 12669 strain, Achromobacter denitrificans ATCC 35699 strain, Devosia riboflavina NBRC 13584 strain, Frateuria aurantia NBRC 3247 strain, Paenibacillus validus NBRC 13635 strain, Brevibacterium butanicum ATCC 21196 strain, Hafnia alvei NBRC 3731 strain, Raoultella planticola JCM 7251 strain, Raoultella terrigena JCM 1687 strain, Rhizobium radiobacter NBRC 13532 strain or Serratia marcesceus NBRC 12648 strain.
5. A method for producing D-lactic acid from glycerol, which comprises:
   culturing in a culture medium containing glycerol, a bacterium belonging to a species selected from the bacterial species group consisting of Arthrobacter nicotianae, Arthrobacter protophormiae, Streptomyces fimicarius, Streptomyces alboflavus and Streptomyces althioticus, and being capable of producing D-lactic acid from glycerol in about 70%e.e. or higher optical purity; or
   contacting cells of the bacterium, processed cells of the bacteriim or immobilized product thereof with glycerol.
6. The method according to item 5, wherein a bacterium belonging to species selected from the bacterial species group consisting of Arthrobacter nicotianae, Arthrobacter protophormiae, Streptomyces fimicarius, Streptomyces alboflavus and Streptomyces althioticus is a bacterium of Arthrobacter nicotianae JCM 1333 strain, Arthrobacter protophormiae JCM 1973 strain, Streptomyces fimicarius ATCC 21900 strain, Streptomyces alboflavus NBRC 13196 strain or Streptomyces althioticus NBRC 15956 strain.
7. A method for producing D-lactic acid from glycerol, which comprises:
   culturing in a culture medium containing glycerol, a bacterium of Escherichia coli ATCC 700926 strain, or
   contacting cells of the bacterium, processed cells of the bacteriim or immobilized product thereof with glycerol.
8. The method according to any one of items 2 to 7, further comprises recovering D-lactic acid from a culture obtained by culturing.
9. A method for producing lactic acid from glycerol, which comprises:
   culturing in a culture medium containing glycerol, a bacterium belonging to a genus selected from the bacterial genus group consisting of Nocardioides and Proteus, and being capable of producing lactic acid from glycerol; or
   contacting cells of the bacterium, processed cells of the bacteriim or immobilized product thereof with glycerol.
10. The method according to item 9, wherein a bacterium belonging to a genus selected from the bacterial genus group consisting of Nocardioides and Proteus is a bacterium of Nocardioides simplex or Proteus vulgaris.
11. The method according to item 10, wherein a bacterium belonging to a genus selected from the bacterial genus group consisting of Nocardioides and Proteus is a bacterium of Nocardioides simplex NBRC 12069 strain or Proteus vulgaris NBRC 3851 strain.
12. A method for producing lactic acid from glycerol, which comprises:
   culturing in a culture medium containing glycerol, a bacterium belonging to a species selected from the bacterial species group consisting of Arthrobacter aurescens, Arthrobacter citreus, Bacillus badius, Bacillus sphaericus, Nocardia uniformis, Streptomyces carnosus and Streptomyces cellulosae, and being capable of producing lactic acid from glycerol; or
   contacting cells of the bacterium, processed cells of the bacteriim or immobilized product thereof with glycerol.
13. The method according to item 12, wherein a bacterium belonging to a species selected from the bacterial species group consisting of Arthrobacter aurescens, Arthrobacter citreus, Bacillus badius, Bacillus sphaericus, Nocardia uniformis, Streptomyces carnosus and Streptomyces cellulosae is a bacterium of Arthrobacter aurescens NBRC 12136 strain, Arthrobacter citrus NBRC 12957 strain. Bacillus badius ATCC 14574 strain. Bacillus sphaericus NBRC 3341 strain, Nocardia uniformis NBRC 13702 strain, Streptomyces carnosus NBRC 13025 strain or Streptomyces cellulosae NBRC 3713 strain.
14. The method according to any one of items 9 to 13, further comprises recovering lactic acid from a culture obtained by culturing.
15. The method according to any one of items 1 to 14, wherein the bacterium is cultured under an aerobic condition.
16. The method according to any one of items 1 to 15, wherein glycerol is originated from Bio Diesel waste.

## Claims

1. A method for producing lactic acid from glycerol, which comprises:
culturing in a culture medium containing glycerol, a bacterium belonging to the genus *Serratia* and being capable of producing D-lactic acid from glycerol in about 70%e.e. or higher optical purity; or
contacting cells of the bacterium, processed cells of the bacterium or
immobilized product thereof with glycerol.

2. A method for producing D-lactic acid from glycerol, which comprises:
culturing in a culture medium containing glycerol, a bacterium belonging to the genus *Serratia* and being capable of producing D-lactic acid from glycerol in about 70%e.e. or higher optical purity; or
contacting cells of the bacterium, processed cells of the bacterium or immobilized product thereof with glycerol.

3. The method according to claim 2, wherein the bacterium belonging to the genus *Serratia* is *Serratia marcesceus.*

4. The method according to claim 3, wherein the bacterium belonging to the genus *Serratia* is *Serratia marcesceus* NBRC 12648 strain.

5. The method according to any one of claims 2 to 4, further comprising recovering D-lactic acid from a culture obtained by culturing.

6. The method according to any one of claim 1 to 5, wherein the bacterium is cultured under an aerobic condition.

7. The method according to any one of claims 1 to 6, wherein glycerol is originated from Bio Diesel waste.
